# EUROPÄISCHE PATENTSCHRIFT

(11) **EP 1 277 484 B1**
(45) Veröffentlichungstag und Bekanntmachung des Hinweises auf die Patenterteilung: **29.09.2004**
(21) Anmeldenummer: 01117508.0
(22) Anmeldetag: 20.07.2001
(51) Int. Cl.: A61L 29/08

(54) **Urologisches Implantat**
Urological implant
Implant urologique

(43) Veröffentlichungstag der Anmeldung: 22.01.2003
(73) Patentinhaber: Hildebrandt, Peter, 90483 Nürnberg (DE)
(72) Erfinder: Hildebrandt, Peter, 90483 Nürnberg (DE)
(74) Vertreter: Schneck, Herbert, Dipl.-Phys., Dr.

(56) Entgegenhaltungen:
- US-A- 5 788 687
- US-B1- 6 193 752
- US-B1- 6 228 393
- HILDEBRANDT P ET AL: "Prevention of surface encrustation of urological implants by coating with inhibitors." BIOMATERIALS. ENGLAND MAR 2001, Bd. 22, Nr. 5, März 2001 (2001-03), Seiten 503-507, XP004227897 ISSN: 0142-9612

## Beschreibung

Die Erfindung betrifft ein urologisches Implantat, insbesondere einen urologischen Katheter, wie z.B. einen Urethralkatheter, suprapubischen Katheter oder Nephrostomiekatheter, mit einem im wesentlichen röhrenförmigen, eigenstabilen Träger. Solche Katheter werden üblicherweise zur Ableitung des Urins verwendet, wenn die normale Ableitung über die harnableitenden Wege nicht mehr möglich ist oder behindert wird. Als weitere Anwendungsbeispiele für die angegebenen urologischen Implantate sind Ureterschienen, Prostata- und Harnleiter-Stents, künstliche Harnleiter oder Blasensysteme zu nennen.

Im folgenden soll der Einfachheit halber bei der Erörterung des Standes der Technik und der Erfindung auf die bereits erwähnten urologischen Katheter beispielhaft eingegangen werden:

Eine große Auswahl an Katheter-Ausgestaltungen und Materialien sind heute verfügbar. Ausgestaltung und Materialien richten sich in der Regel nach dem Einsatzort des Katheters und der verwendeten Implantationstechnik. So sind beispielsweise urologische Katheter mit einem Ballon am im Körper befindlichen Ende üblich. Nachdem der Katheter plaziert wurde, wird der Ballon mit Flüssigkeit gefüllt und dehnt sich dadurch z.B. in der Harnblase oder dem Nierenbecken liegend auf. Durch den gefüllten Ballon wird ein unbeabsichtigtes Herausrutschen des Katheters verhindert. Zum Entfernen des Katheters wird der Ballon wieder entleert. Andere urologische Katheter verfügen z.B. über eine Schlinge am im Körper befindlichen Ende, die bei der Implantation durch einen im Lumen des Katheters befindlichen Draht gestreckt wird. Nach der Plazierung wird der Draht entfernt, die Schlinge bildet sich wieder aus und hält so den Katheter in seiner Position.

Als schwerwiegendes Problem tritt bei allen bisher eingesetzten und getesteten Materialien für urologische Katheter zum Teil bereits nach Liegezeiten von 24 Stunden die Besiedlung mit Bakterien ein. Dies führt zu einer Harnwegsinfektion, welche die oft ohnehin geschwächten Patienten zusätzlich belastet. Eine Behandlung mit Antibiotika unter Inkaufnahme derer Nebenwirkungen wird notwendig. Bei längeren Liegezeiten tritt zudem Kristallbildung an der Katheter-Oberfläche auf, was zum Verschluß des Katheters und damit zum Verlust der Drainagefunktion führt.

Ein weiteres Problem stellt der hohe Reibungskoeffizient der bei urologischen Kathetern verwendeten Materialien dar. Dadurch wird das Einbringen von Kathetern und Ureterschienen in die entsprechenden Körperhöhlungen deutlich erschwert und in schwierigen Fällen sogar unmöglich gemacht. Zusätzlich haben die Patienten zum Teil starke Schmerzen und es kann zur Traumatisierung des Gewebes durch die Reibung am Katheter kommen.

Ein Ansatz aus dem Stand der Technik zur Lösung der Bakterienbesiedlungsproblematik ist der US 6 228 393 A zu entnehmen. In dieser Druckschrift ist eine Hydrogelbeschichtung offenbart, die Wirkstoffe wie beispielsweise Antibiotika freigibt. Dadurch soll die Bildung eines Bakterienfilms an der Oberfläche verhindert werden. Einen vergleichbaren Ansatz wählt die US 5 788 687 A. Hier soll bei Überschreitung eines bestimmten, durch eine Infektion erhöhten pH-Werts die Beschichtung einen antibakteriellen Wirkstoff freigeben.

Die dem Stand der Technik entnommenen Ansätze verfolgen eine Eindämmung der bakteriellen Besiedlung der Oberfläche durch die Freigabe von Wirkstoffen. Dabei wird jedoch ein Reservoir an Wirkstoffen benötigt, das zwangsläufig beschränkt ist. Ist dieses Reservoir aufgebraucht, so nimmt die Oberfläche wieder ihre ursprünglichen Eigenschaften bezüglich der Anfälligkeit für bakterielle Besiedlung an. Dies läßt die Anwendung bei längeren Liegezeiten als wenig sinnvoll erscheinen. Damit ist die mit diesem Stand der Technik erreichbare Wirkung nach wie vor verbesserungsbedürftig. Auf die Lösung dieser Problematik zielt die vorliegende Erfindung ab.

Glykosaminoglykane, zu denen beispielsweise Chondroitinsulfat und Hyaluronsäure zählen, sind Stoffe, die natürlich im Urin vorkommen. Deren antimikrobielle und kristallisationsinhibierende Eigenschaften wurden bereits erforscht und nachgewiesen. So hemmen diese Stoffe z.B. im Speicherorgan Harnblase die Entwicklung von Infektionen und Kristallen (Blasensteinen), indem sie Bakterien und Kristallite einschließen. Die nun an ihrer Oberfläche vollständig mit Glykosaminoglykanen bedeckten Bakterien und Kristallite sind chemisch maskiert und stellen so bis zu ihrer Ausscheidung keine Gefahr für den menschlichen Körper dar. Eine Implantatoberfläche die ausreichend mit Glykosaminoglykanen durchsetzt ist stellt sich nun chemisch ebenfalls maskiert dar, Bakterien- und Kristallwachstum treten dort primär nicht auf. Auf diesem Effekt beruht das in der DE 197 29 279 A 1 offenbarte urologische Implantat, bei dem an der Trägeroberfläche über eine Spacer-Lage eine Inhibitor-Lage aus einem Glykosaminoglykan gebunden ist.

Vor dem Hintergrund dieser biomedizinischen Grundlagen schlägt nun die Erfindung laut Kennzeichungsteil des Anspruches 1 zur Lösung der eingangs geschilderten Problematik ein urologisches Implantat vor, an dessen Oberfläche ein Hydrogel gebunden ist, in welches ein Glykosaminoglykan kovalent eingebaut ist. Durch die kovalente Einbindung der Glykosaminoglykane erfolgt keine Wirkstofffreigabe und die erwünschten antimikrobiellen und kristallisationsinhibierenden Eigenschaften der Beschichtung haben Bestand. Die Verwendung eines Hydrogels als Grundstoff für die Beschichtung löst zusätzlich das eingangs beschriebene Problem des hohen Reibungskoeffizienten. Hydrogels haben durch ihren hohen Wassergehalt von z.T. über 60% eine glitschige Oberfläche. Damit wird die Implantation eines erfindungsgemäß ausgestatteten Implantats deutlich erleichtert. Die Beschichtung von Kathetern mit hydrophilen Stoffen zur Verringerung des Reibungskoeffizienten ist grundsätzlich aus dem Stand der Technik, wie beispielsweise aus der US 5 041 100 A bekannt.

Gemäß bevorzugten Ausführungsformen kann der Träger einerseits aus einem Polymer, wie Silikon, Polyurethan oder dergleichen oder andererseits aus Tantal, einer Titanlegierung, medizinischem Stahl oder pyrolytischem Kohlenstoff bestehen. Auch eine Ausbildung aus einem Metallgerüst mit einer Polymer-Beschichtung ist möglich.

Das Hydrogel wird vorzugsweise aus Polyvinylalkohol, Glutaraldehyd, Salzsäure oder Magnesiumchlorid als Katalysator, Wasser und einem Glykosaminoglykan gebildet. Auch die Verwendung von Polyethylenglykol ist möglich. In das vernetzte Hydrogel ist das Glykosaminoglykan mit Hilfe einer Schiff-Base über eine kovalente Bindung eingebaut. Zur Bindung des Hydrogels an die Oberfläche des Trägers wird diese vorzugsweise mit Salzsäure oder Schwefelsäure geätzt. Dadurch bilden sich an der Oberfläche Hydroxylgruppen, welche die Anbindung des Hydrogels ermöglichen. Je nach Material des Trägers kommen auch andere Verfahren zur Aktivierung der Oberfläche, wie beispielsweise Modifikationen im Plasma in Frage.

Zusammenfassend haben experimentelle Untersuchungen mit erfindungsgemäß ausgerüsteten Kathetern, bei denen ein Polyvinylalkohol-Hydrogel auf einem Polyurethan Träger eingesetzt wird, eine signifikante Verringerung der Bakterienbesiedlung ergeben. So konnte bei in vitro Versuchen durch Eintauchen entsprechender Proben in Bakteriensuspensionen (Escherichia coli, Staphylococcus aureus, Proteus mirabilis, Pseudomonas aeruginosa und Staphylococcus epidermidis) über mehrere Tage eine Reduktion der Bakterienbesiedlung auf ein Zehntel bis hin z.T. auf Null nachgewiesen werden. Weiterhin wurde im gequollenen Zustand der Hydrogelbeschichtung ein um Faktor 8 verringerter Reibungskoeffizient gemessen. Proben mit Hydrogelbeschichtung wurden zudem mit Hilfe einer Spurenelementanalyse (Ionenabgabetest) auf die Freigabe der eingebundenen Glykosaminoglykane untersucht. Dazu wurden die Proben gut gespült und gequollen. Nach einer anschließenden 168-stündigen ununterbrochenen Umspülung in Kochsalzlösung wurde keine Freigabe des Glykosaminoglykans gemessen. Hydrogels die zur Wirkstofffreigabe verwendet werden zeigen bei vergleichbaren Tests eine Freigabe von z.T. mehr als 70% (m/m) des ursprünglich enthaltenen Wirkstoffs.

Die beigefügten Zeichnungen erläutern die Merkmale, Einzelheiten und Vorteile des Erfindungsgegenstandes näher. Es zeigen:
- Fig. 1: einen ausschnittsweisen Schnitt durch ein Implantat,
- Fig. 2: eine chemische Strukturformel des kovalent gebundenen Glykosaminoglykans, und
- Fig. 3: eine perspektivische, ausschnittsweise Darstellung eines Katheters.

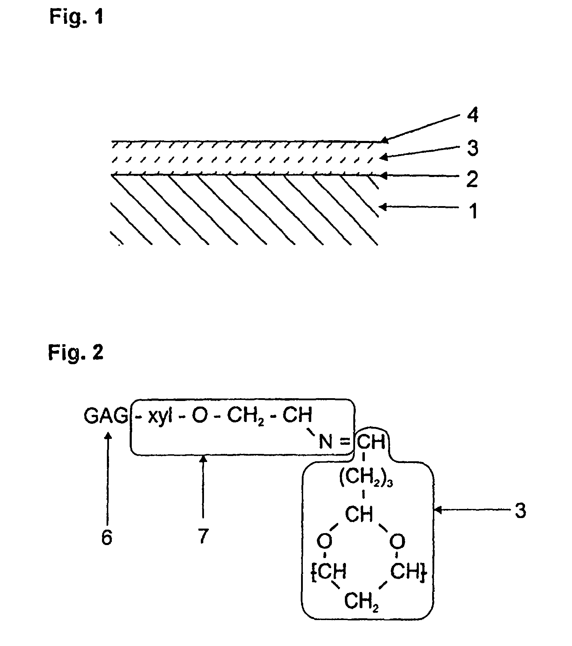

Fig. 1 zeigt einen Träger 1 für ein urologisches Implantat, der beispielsweise aus Polyurethan besteht. An dessen Oberfläche 2 ist eine Schicht 3 eines Hydrogels gebunden, so daß nach außen die gut gleitfähige Oberfläche 4 der Hydrogelschicht 3 frei liegt.

In Fig. 2 ist durch die dort dargestellte Strukturformel die Einbindung des Glykosaminoglykans (GAG) 6 über eine kovalente Bindung 7 in das Hydrogel 3 erkennbar.

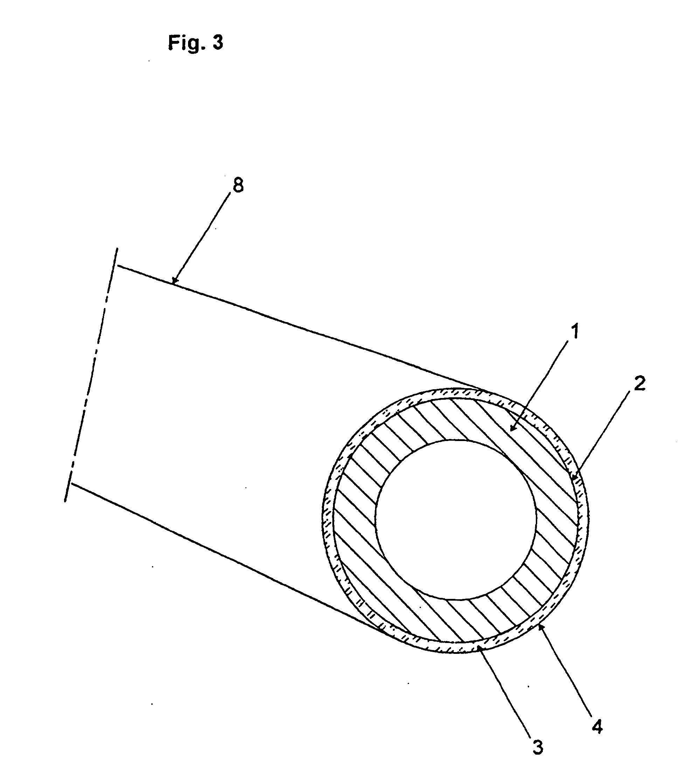

Fig. 3 zeigt einen urologischen Katheter 8 mit einem röhrenförmigen Träger 1 aus Polyurethan und der erörterten Hydrogelschicht 3 mit GAG 6.

Nachfolgend wird in Form einer Auflistung einzelner Verfahrensschritte beispielhaft ein Beschichtungsverfahren auf einer Polyurethanoberfläche wiedergegeben:
- 700mg Polyvinylalkohol (125'000 g/Mol) in 6,3ml destilliertes Wasser einrühren
- 1ml Glutaraldehyd (50% in Wasser), 2ml destilliertes Wasser und 20µl Salzsäure (1 molar) mischen und in die Polyvinylalkohol-Mischung gut einrühren
- 0,5ml einer Glykosaminoglykan-Lösung (25'000 IU/ml in Wasser) einrühren
- die Polyurethan-Probe 1 Minute in 37%-iger Salzsäure ätzen und anschließend in destilliertem Wasser spülen
- die geätzte Polyurethan-Probe in das Hydrogel eintauchen und wenden
- die mit Hydrogel benetzte Probe für 90 Minuten bei 60°C trocknen
- Probe gründlich in physiologischer Kochsalzlösung spülen und an Luft trocknen

## Patentansprüche

1. Urologisches Implantat, insbesondere urologischer Katheter (8), mit einem vorzugsweise im wesentlichen röhrenförmigen, eigenstabilen Träger (1),
**gekennzeichnet durch**
eine an der Trägeroberfläche (2) gebundene Hydrogelschicht (3) in welche ein Glykosaminoglykan (6) über eine kovalente Bindung (7) eingebaut ist.

2. Implantat nach Anspruch 1, **dadurch gekennzeichnet, daß** der Träger (1) aus einem Polymer, insbesondere Silikon oder Polyurethan besteht.

3. Implantat nach Anspruch 1, **dadurch gekennzeichnet, daß** der Träger (1) aus einem eigenstabilen Gerüst, insbesondere aus Metall mit einer Beschichtung aus einem Polymer, insbesondere Silikon oder Polyurethan besteht.

4. Implantat nach Anspruch 1, **dadurch gekennzeichnet, daß** der Träger (1) aus Tantal, einer Titanlegierung, medizinischem Stahl oder pyrolytischem Kohlenstoff besteht.

5. Implantat nach einem der Ansprüche 1 bis 4, **dadurch gekennzeichnet, daß** das Hydrogel (3) aus einer Polyvinylalkohol-Verbindung gebildet ist.

6. Implantat nach einem der Ansprüche 1 bis 4, **dadurch gekennzeichnet, daß** das Hydrogel (3) aus einer Polyethylenglykol-Verbindung gebildet ist.

7. Implantat nach einem der Ansprüche 1 bis 4, **dadurch gekennzeichnet, daß** das Hydrogel (3) auf der Basis von Glutaraldehyd gebildet ist.

8. Implantat nach einem der Ansprüche 1 bis 4, **dadurch gekennzeichnet, daß** das Hydrogel (3) auf der Basis von Formaldehyd gebildet ist.

9. Implantat nach einem der Ansprüche 1 bis 4, **dadurch gekennzeichnet, daß** das Hydrogel (3) auf der Basis von Salzsäure oder Magnesiumchlorid gebildet ist.

10. Implantat nach einem der Ansprüche 1 bis 9, **dadurch gekennzeichnet, daß** die Bindung zwischen Hydrogel (3) und Trägeroberfläche (2) mit Hilfe einer Säure, insbesondere Salzsäure oder Schwefelsäure gebildet ist.

11. Implantat nach einem der Ansprüche 1 bis 9, **dadurch gekennzeichnet, daß** die Bindung zwischen Hydrogel (3) und Trägeroberfläche (2) mit Hilfe einer Plasamabehandlung der Trägeroberfläche (2) gebildet ist.

## Claims

1. Urological implant, in particular a urological catheter (8), comprising a preferably substantially tubular, inherently stable carrier (I),
**characterised by**
a hydrogel layer (3) bound on the carrier surface (2), into which a glycosaminoglycan (6) is incorporated via a covalent bond (7).

2. Implant according to claim 1, **characterised in that** the carrier (1) comprises a polymer, in particular silicone or polyurethane.

3. Implant according to claim 1, **characterised in that** the carrier (1) comprises an inherently stable skeleton, in particular made of metal with a polymer, in particular silicone or polyurethane, coating.

4. Implant according to claim 1, **characterised in that** the carrier (1) is made of tantalum, a titanium alloy, medical steel or pyrolytic carbon.

5. Implant according to any one of claims 1 to 4, **characterised in that** the hydrogel (3) is formed from a polyvinyl alcohol compound.

6. Implant according to any one of claims 1 to 4, **characterised in that** the hydrogel (3) is formed from a polyethylene glycol compound.

7. Implant according to any one of claims 1 to 4, **characterised in that** the hydrogel (3) is formed on the basis of glutaraldehyde.

8. Implant according to any one of claims 1 to 4, **characterised in that** the hydrogel (3) is formed on the basis of formaldeyde.

9. Implant according to any one of claims 1 to 4, **characterised in that** the hydrogel (3) is formed on the basis of hydrochloric acid or magnesium chloride.

10. Implant according to any one of claims 1 to 9, **characterised in that** the bond between the hydrogel (3) and the carrier surface (2) is formed using an acid, in particular hydrochloric acid or sulphuric acid.

11. Implant according to any one of claims 1 to 9, **characterised in that** the bond between the hydrogel (3) and the carrier surface (2) is formed by means of a plasma treatment of the carrier surface (2).

## Revendications

1. Implant urologique, en particulier cathéter urologique (8), comprenant un support (1) de préférence sensiblement tubulaire, auto-stable, **caractérisé par** une couche d'hydrogel (3) liée sur la surface du support (2), dans laquelle est intégré un glycosaminoglycane (6) par une liaison covalente (7).

2. Implant selon la revendication 1, **caractérisé en ce que** le support (1) est réalisé dans un polymère, en particulier la silicone ou le polyuréthanne.

3. Implant selon la revendication 1, **caractérisé en ce que** le support (1) est formé par une structure auto-stable, en particulier en métal avec un revêtement en polymère, en particulier en silicone ou polyuréthanne.

4. selon la revendication 1, **caractérisé en ce que** le support (1) est réalisé en tantale, en alliage de titane, en acier à usage médical ou en carbone pyrolytique.

5. Implant selon l'une quelconque des revendications 1 à 4, **caractérisé en ce que** l'hydrogel (3) est formé par un composé à base d'alcool polyvinylique.

6. Implant selon l'une quelconque des revendications 1 à 4, **caractérisé en ce que** l'hydrogel (3) est formé par un composé à base de polyéthylène glycol.

7. Implant selon l'une quelconque des revendications 1 à 4, **caractérisé en ce que** l'hydrogel (3) est formé sur la base d'un aldéhyde glutarique.

8. Implant selon l'une quelconque des revendications 1 à 4, **caractérisé en ce que** l'hydrogel (3) est formé sur la base d'un formaldéhyde.

9. Implant selon l'une quelconque des revendications 1 à 4, **caractérisé en ce que** l'hydrogel (3) est formé à base d'acide chlorhydrique ou de chlorure de magnésium.

10. Implant selon l'une quelconque des revendications 1 à 9, **caractérisé en ce que** la liaison entre l'hydrogel (3) et la surface du support (2) est formée au moyen d'un acide, en particulier l'acide chlorhydrique ou l'acide sulfurique.

11. Implant selon l'une quelconque des revendications 1 à 9, **caractérisé en ce que** la liaison entre l'hydrogel (3) et la surface du support (2) est formée au moyen d'un traitement par plasma de la surface du support (2).
